(19) Europäisches Patentamt / European Patent Office / Office européen des brevets

(11) **EP 4 796 123 A1**

(12) **EUROPEAN PATENT APPLICATION**
published in accordance with Art. 153(4) EPC


(43) Date of publication: **26.08.2026 Bulletin 2026/35**

(21) Application number: **24879089.1**

(22) Date of filing: **17.10.2024**

(51) International Patent Classification (IPC): ***A61K 39/395*** $^{(2006.01)}$ ***A61P 35/00*** $^{(2006.01)}$

(52) Cooperative Patent Classification (CPC): **A61K 39/395; A61P 35/00**

(86) International application number: **PCT/CN2024/125546**

(87) International publication number: **WO 2025/082449 (24.04.2025 Gazette 2025/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.10.2023 CN 202311365208**
**07.02.2024 CN 202410176715**
**30.09.2024 CN 202411393577**



(71) Applicants:
- **Jiangsu Hansoh Pharmaceutical Group Co., Ltd. Lianyungang, Jiangsu 222047 (CN)**
- **Shanghai Hansoh Biomedical Company Limited Pudong New Area, Shanghai 201203 (CN)**
- **Changzhou Hansoh Pharmaceutical Company Limited Changzhou, Jiangsu 213001 (CN)**

(72) Inventors:
- **SUN, Danni Shanghai 201203 (CN)**
- **ZHOU, Yuanfeng Shanghai 201203 (CN)**
- **XU, Min Shanghai 201203 (CN)**
- **LIU, Junhao Shanghai 201203 (CN)**
- **QIU, Pengchao Shanghai 201203 (CN)**

(74) Representative: **Hanby, Abigail Rose**
**GSK**
**Legal & Compliance - Global Patents**
**79 New Oxford Street**
**London WC1A 1DG (GB)**


# (54) COMBINED USE OF ANTIBODY-DRUG CONJUGATE AND IMMUNE CHECKPOINT INHIBITOR


(57) Provided is a use of an antibody-drug conjugate alone or in combination in the preparation of drugs for preventing and/or treating cancer. Specifically, provided is a use of an antibody-drug conjugate or a pharmaceutically acceptable salt, metabolite or solvent compound thereof alone or in combination with an immune checkpoint inhibitor in the preparation of drugs for preventing and/or treating cancer.

SJSA-1 model
Change in body weight (%)
Mean ± SEM
20
15
10
5
0
-5
-10
-15
0
3
6
9
12
15
18
21
24
Time after dosing (days)
Vehicle
Drug A
Drug B
Drug C
Drug A + Drug B
Drug A + Drug C


Figure 5




Processed by Luminess, 75001 PARIS (FR)

## Description

### Technical Field

The present application belongs to the field of medicine, and relates to use of an antibody-drug conjugate alone or in combination in the preparation of a medication for preventing and/or treating cancer. Specifically, the present invention provides use of an antibody-drug conjugate or a pharmaceutically acceptable salt, metabolite, or solvate compound thereof alone or in combination with an immune checkpoint inhibitor in the preparation of a medication for preventing and/or treating cancer.

### Background Art

Antibody-drug conjugates (antibody-drug conjugate, ADC) are a class of targeted biologic agents in which a cytotoxic drug is linked to a monoclonal antibody through a linker, using the monoclonal antibody as a carrier to efficiently transport the small-molecule cytotoxic drug to target tumor cells in a targeted manner. Tumor-specific antibodies enable ADC drugs to selectively deliver small-molecule cytotoxic drugs, while reducing the off-target effects of the small-molecule cytotoxic drugs and retaining their antitumor properties, thereby effectively improving the benefit to risk ratio of antitumor treatment. B7 homologous protein 3 (B7-H3, also known as CD276, and referred to herein as "B7-H3" or "B7H3") is a type I transmembrane glycoprotein of the immunoglobulin superfamily and is a member of the B7 immune costimulatory and coinhibitory family. It is overexpressed in a variety of solid tumors such as lung cancer, prostate cancer, esophageal cancer, head and neck squamous cell carcinoma, osteosarcoma, bladder cancer, and melanoma, but has limited expression in normal tissues, and therefore B7-H3 is an ideal target for ADC drugs.

Immune checkpoint inhibitors (immune checkpoint inhibitors, ICIs) block the binding of immune checkpoints to their ligands, relieve the immunosuppression caused by immune checkpoints, thereby reactivating immune cells to exert antitumor effects. At present, multiple ICIs have achieved remarkable effects in antitumor clinical applications, and represent a breakthrough in tumor therapy. Inhibitors targeting cytotoxic T lymphocyte-associated antigen-4 (cytotoxic T lymphocyte-associated antigen-4, CTLA-4), programmed death-1 receptor (programmed death-1, PD-1), and programmed death-ligand 1 (programmed death-ligand 1, PD-L1) have been successfully used in the clinical treatment of a variety of malignant tumors, among which PD-1/PD-L1 antibody drugs have a broad anticancer spectrum and show durable and potent efficacy in some tumor patients, and 10 PD-1 monoclonal antibodies and 3 PD-L1 monoclonal antibodies have been approved for the clinical treatment of 11 cancer types such as non-small cell lung cancer, melanoma, head and neck cancer, colorectal cancer, and gastric cancer. Although ICIs have significant antitumor activity in multiple types of malignant tumors, their response rate in most tumors is relatively low, and new regimens need to be developed to improve the response rate to ICI treatment.

Conventional osteosarcoma (hereinafter referred to as osteosarcoma) is a common malignant bone tumor, accounting for 35% of all malignant bone tumors. Conventional osteosarcoma occurs frequently in adolescents, with a median age of onset of 20 years, and is the most common primary malignant bone tumor in children and adolescents. Approximately 10% to 15% of newly diagnosed osteosarcoma patients present with distant metastasis, the 5-year survival rate of patients with localized osteosarcoma is about 60%, whereas the 5-year survival rate of patients with advanced-stage (recurrent or metastatic) osteosarcoma is only about 20%. The first-line standard chemotherapy regimen for osteosarcoma is a regimen containing methotrexate, doxorubicin, cisplatin, and ifosfamide. For osteosarcoma patients who cannot undergo curative surgical treatment, after failure of standard chemotherapy, effective treatment means are lacking, and there is still no drug or other treatment option that can demonstrate significant survival benefit for such patients. At present, no second-line treatment regimen has been approved, and the existing treatment methods have relatively great hematologic toxicity and many adverse reactions. Therefore, improving the efficacy of second-line osteosarcoma treatment and prolonging the survival period of patients is one of the objectives of current clinical research, and new treatment methods are urgently needed to meet the treatment needs of patients.

The preferred combination therapy of the present invention exhibits an effect higher than that of each treatment used alone, and while improving efficacy, has fewer side effects associated with combination therapy, promoting the maximization of survival benefit for patients receiving immunotherapy.

### Summary of the Invention

The present disclosure provides use of a combination of an antibody-drug conjugate and an immune checkpoint inhibitor in the preparation of a drug for treating cancer, wherein the structure of the antibody-drug conjugate is as shown in Formula (I):

In the formula, n is a non-zero integer or decimal from 1 to 10, preferably a decimal or integer between 1 and 8, preferably a decimal or integer between 2 and 8, more preferably 3 to 8, which may be an integer or may be a decimal, and more preferably 4.1.

Pc is an anti-B7H3 antibody or an antigen-binding fragment thereof.

In some embodiments, the anti-B7H3 antibody or an antigen-binding fragment thereof comprises: heavy-chain HCDR1, HCDR2, and HCDR3 as shown by the amino acid sequences of SEQ ID NO: 01, 02, and 03, respectively, and light-chain LCDR1, LCDR2, and LCDR3 as shown by the amino acid sequences of SEQ ID NO: 04, 05, and 06, respectively.

In the present invention, the amino acid sequences of the CDRs listed above are all shown according to the Kabat definition rules. However, it is well known to those skilled in the art that in this field, the CDRs of an antibody can be defined by various methods. Although the claimed protection scope of the present invention is based on the sequences shown according to the Kabat definition rules, the amino acid sequences corresponding to other CDR definition rules should also fall within the protection scope of the present invention.

The above-mentioned respective CDR sequences are as shown in the following table:

Table 1 Respective heavy-chain and light-chain CDR sequences

| HCDR1 | SSAMH SEQ ID NO: 01 | LCDR1 | GLSSGSVSTSHYPS SEQ ID NO: 04 |
|---|---|---|---|
| HCDR2 | VISYDGSNKYYVDSVKG SEQ ID NO: 02 | LCDR2 | NTNTRSS SEQ ID NO: 05 |
| HCDR3 | SARLYASFDY SEQ ID NO: 03 | LCDR3 | AIHVDRDIWV SEQ ID NO: 06 |
| Note: The CDR sequences are determined according to the Kabat definition. | | | |

Preferably, the anti-B7H3 antibody or the antigen-binding fragment thereof is selected from a humanized antibody or a fragment thereof.

In some optional embodiments, the anti-B7H3 antibody or an antigen-binding fragment thereof as described in the present application is an antibody fragment selected from the group consisting of Fab, Fab'-SH, Fv, scFv, and $(Fab')_2$ fragments.

In some optional embodiments, the anti-B7H3 antibody or an antigen-binding fragment thereof as described in the present application comprises a heavy-chain constant region of the human IgG1, IgG2, IgG3, or IgG4 isotype, preferably comprises a heavy-chain constant region of the IgG1 or IgG4 isotype.

In other optional embodiments, the anti-B7H3 antibody or the antigen-binding fragment thereof comprises κ or λ light-chain constant region.

Further, preferably, the heavy-chain variable region sequence of the anti-B7H3 antibody or the antigen-binding fragment thereof is the sequence as shown in SEQ ID NO: 07 or a variant thereof, and the light-chain variable region sequence is the sequence as shown in SEQ ID NO: 08 or a variant thereof.

The sequences of the heavy-chain and light-chain variable regions of the aforesaid anti-B7H3 antibody or the antigen-binding fragment thereof are as follows:

Heavy-chain variable region sequence

*QVQLVQSGGGVVQPGTSLRLSCAASGFIFS*SSAMH*WVRQAPGKGLEWVA*VISYDGSNKY
YVDSVKG*RFTISRDNSKNTLYLQMNSLRAEDTAVYYCAR*SARLYASFDY*WGQGALVTVSS*

SEQ ID NO: 07

Light-chain variable region sequence

*DTVVTQEPSFSVSPGGTVTLTC*GLSSGSVSTSHYPS*WYQQTPGQAPRMLIY*NTNTRSS*GV PDRFSGSILGNKAALTITGAQADDESDYYC*AIHVDRDIWV*FGGGTKLTVL*

SEQ ID NO: 08

Note: The order is FR1-CDR1-FR2-CDR2-FR3-CDR3-FR4, and in the sequence the italicized are FR sequences, and the underlined is CDR sequences, wherein the CDR sequences are derived from the Kabat definition rules.

Further, preferably, the heavy-chain sequence of the anti-B7H3 antibody or an antigen-binding fragment thereof is the sequence as shown in SEQ ID NO: 09 or a variant thereof, and the light-chain sequence is the sequence as shown in SEQ ID NO: 10 or a variant thereof.

The sequences of the heavy chain and light chain of the aforesaid anti-B7H3 antibody or an antigen-binding fragment thereof are as follows:

Heavy-chain (IgG1) amino acid sequence: (SEQ ID NO: 09)

QVQLVQSGGGVVQPGTSLRLSCAASGFIFSSSAMHWVRQAPGKGLEWVAVISYD GSNKYYVDSVKGRFTISRDNSKNTLYLQMNSLRAEDTAVYYCARSARLYASFDYWG QGALVTVSSASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVEPKSCDKTH TCPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKA KGQPREPQVYTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSPGK

Light-chain (λ) amino acid sequence: (SEQ ID NO: 10)

DTVVTQEPSFSVSPGGTVTLTCGLSSGSVSTSHYPSWYQQTPGQAPRMLIYNTNT RSSGVPDRFSGSILGNKAALTITGAQADDESDYYCAIHVDRDIWVFGGGTKLTVLGQP KANPTVTLFPPSSEELQANKATLVCLISDFYPGAVTVAWKADGSPVKAGVETTKPSKQ SNNKYAASSYLSLTPEQWKSHRSYSCQVTHEGSTVEKTVAPTEC

In some embodiments, the immune checkpoint inhibitor is selected from an antibody targeting PD-1, PD-L1, CTLA-4, LAG-3, TIM-3, TIGIT, BTLA, A2aR, B7-H3, or B7-H4, or an antigen-binding fragment thereof. Preferably, an antibody targeting PD-1 or PD-L1, or an antigen-binding fragment thereof.

In some embodiments, the immune checkpoint inhibitor is selected from toripalimab (Toripalimab), sintilimab (Sintilimab), camrelizumab (Camrelizumab), dostarlimab (Dostarlimab), tislelizumab (Tislelizumab), zimberelimab (Zimberelimab), penpulimab (Penpulimab), serplulimab (Serplulimab), pucotenlimab (Pucotenlimab), pembrolizumab (Pembrolizumab), nivolumab (Nivolumab), sugemalimab (Sugemalimab), envafolimab (Envafolimab), adebrelimab (Adebrelimab), atezolizumab (Atezolizumab), durvalumab (Durvalumab), ipilimumab (Ipilimumab), and candonilimab antibody (Candonilimab), preferably camrelizumab (Camrelizumab) and adebrelimab (Adebrelimab).

The sequences of the heavy chain and light chain of the aforesaid camrelizumab are as follows:

Heavy-chain amino acid sequence: (SEQ ID NO: 11)

EVQLVESGGGLVQPGGSLRLSCAASGFTFSSYMMSWVRQAPGKGLEWVATISGG GANTYYPDSVKGRFTISRDNAKNSLYLQMNSLRAEDTAVYYCARQLYYFDYWGQGT TVTVSSASTKGPSVFPLAPCSRSTSESTAALGCLVKDYFPEPVTVSWNSGALTSGVHTF PAVLQSSGLYSLSSVVTVPSSSLGTKTYTCNVDHKPSNTKVDKRVESKYGPPCPPCPAP EFLGGPSVFLFPPKPKDTLMISRTPEVTCVVVDVSQEDPEVQFNWYVDGVEVHNAKT KPREEQFNSTYRVVSVLTVLHQDWLNGKEYKCKVSNKGLPSSIEKTISKAKGQPREP QVYTLPPSQEEMTKNQVSLTCLVKGFYPSDIAVEWESNGQPENNYKTTPPVLDSDGSF FLYSRLTVDKSRWQEGNVFSCSVMHEALHNHYTQKSLSLSLGK

Light-chain amino acid sequence: (SEQ ID NO: 12)

DIQMTQSPSSLSASVGDRVTITCLASQTIGTWLTWYQQKPGKAPKLLIYTATSLA DGVPSRFSGSGSGTDFTLTISSLQPEDFATYYCQQVYSIPWTFGGGTKVEIKRTVAAPS VFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGEC

The sequences of the heavy chain and light chain of the aforesaid adebrelimab are as follows:

Heavy-chain amino acid sequence: (SEQ ID NO: 13)

QVQLVQSGAEVKKPGASVKVSCKASGYTFTSYWMHWVRQAPGQGLEWMGRI GPNSGFTSYNEKFKNRVTMTRDTSTSTVYMELSSLRSEDTAVYYCARGGSSYDYFDY WGQGTTVTVSSASTKGPSVFPLAPCSRSTSESTAALGCLVKDYFPEPVTVSWNSGALT SGVHTFPAVLQSSGLYSLSSVVTVPSSSLGTKTYTCNVDHKPSNTKVDKRVESKYGPP CPPCPAPEAAGGPSVFLFPPKPKDTLMISRTPEVTCVVVDVSQEDPEVQFNWYVDGVE VHNAKTKPREEQFNSTYRVVSVLTVLHQDWLNGKEYKCKVSNKGLPSSIEKTISKAK GQPREPQVYTLPPSQEEMTKNQVSLTCLVKGFYPSDIAVEWESNGQPENNYKTTPPVL DSDGSFFLYSRLT VDKSRWQEGN VFSCSVMHEA LHNHYTQKSL SLSLGK

Light-chain amino acid sequence: (SEQ ID NO: 14)

DIVLTQSPASLAVSPGQRATITCRASESVSIHGTHLMHWYQQKPGQPPKLLIYAAS NLESGVPARFSGSGSGTDFTLTINPVEAEDTANYYCQQSFEDPLTFGQGTKLEIKRTVA APSVFIFPPSDEQLKSGTASVVCLLNNFYPREAKVQWKVDNALQSGNSQESVTEQDS KDSTYSLSSTLTLSKADYEKHKVYACEV THQGLSSPVT KSFNRGEC

Dostarlimab is a PD-1-blocking IgG4 humanized monoclonal antibody, the trade name is JEMPERLI, and the main structure and functions of dostarlimab have been described in WO 2014/179664, WO 2018/085468, and WO 2018/129559.

The heavy-chain and light-chain sequences of dostarlimab are as follows:

Heavy-chain amino acid sequence: (SEQ ID NO: 15)

EVQLLESGGGLVQPGGSLRLSCAASGFTFSSYDMSWVRQAPGKGLEWV STISGGGSYTYYQDSVKGRFTISRDNSKNTLYLQMNSLRAEDTAVYYCASPYY AMDYWGQGTTVTVSSASTKGPSVFPLAPCSRSTSESTAALGCLVKDYFPEPVT

VSWNSGALTSGVHTFPAVLQSSGLYSLSSVVTVPSSSLGTKTYTCNVDHKPSN
TKVDKRVESKYGPPCPPCPAPEFLGGPSVFLFPPKPKDTLMISRTPEVTCVVVD
VSQEDPEVQFNWYVDGVEVHNAKTKPREEQFNSTYRVVSVLTVLHQDWLNG
KEYKCKVSNKGLPSSIEKTISKAKGQPREPQVYTLPPSQEEMTKNQVSLTCLV
KGFYPSDIAVEWESNGQPENNYKTTPPVLDSDGSFFLYSRLTVDKSRWQEGNV
FSCSVMHEALHNHYTQKSLSLSLGK

Light-chain amino acid sequence: (SEQ ID NO: 16)

DIQLTQSPSFLSAYVGDRVTITCKASQDVGTAVAWYQQKPGKAPKLLIYW
ASTLHTGVPSRFSGSGSGTEFTLTISSLQPEDFATYYCQHYSSYPWTFGQGTKL
EIKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFN
RGEC

In some embodiments, dostarlimab or a biosimilar thereof is administered to a patient in need, at a dose of 500 mg, once every three weeks (Q3W).

In some embodiments, dostarlimab or a biosimilar thereof is administered to a patient in need, at a dose of 500 mg, once every three weeks (Q3W), for 4-6 cycles, and then 1000 mg of dostarlimab or a biosimilar thereof is administered, once every 6 weeks (Q6W).

Pembrolizumab is a PD-1-blocking IgG4 humanized monoclonal antibody, and the trade name is KEYTRUDA.

The heavy-chain and light-chain sequences of pembrolizumab are as follows:

Heavy-chain amino acid sequence: (SEQ ID NO: 17)

QVQLVQSGVEVKKPGASVKVSCKASGYTFTNYYMYWVRQAPGQGLEW
MGGINPSNGGTNFNEKFKNRVTLTTDSSTTTAYMELKSLQFDDTAVYYCARR
DYRFDMGFDYWGQGTTVTVSSASTKGPSVFPLAPCSRSTSESTAALGCLVKD
YFPEPVTVSWNSGALTSGVHTFPAVLQSSGLYSLSSVVTVPSSSLGTKTYTCNV
DHKPSNTKVDKRVESKYGPPCPPCPAPEFLGGPSVFLFPPKPKDTLMISRTPEV
TCVVVDVSQEDPEVQFNWYVDGVEVHNAKTKPREEQFNSTYRVVSVLTVLH
QDWLNGKEYKCKVSNKGLPSSIEKTISKAKGQPREPQVYTLPPSQEEMTKNQ
VSLTCLVKGFYPSDIAVEWESNGQPENNYKTTPPVLDSDGSFFLYSRLTVDKSR
WQEGNVFSCSVMHEALHNHYTQKSLSLSLGK

Light-chain amino acid sequence: (SEQ ID NO: 18)

EIVLTQSPATLSLSPGERATLSCRASKGVSTSGYSYLHWYQQKPGQAPRLL
IYLASYLESGVPARFSGSGSGTDFTLTISSLEPEDFAVYYCQHSRDLPLTFGGGT

KVEIKRTVAAPSVFIFPPSDEQLKSGTASVVCLLNNFYPREAKVQWKVDNALQ
SGNSQESVTEQDSKDSTYSLSSTLTLSKADYEKHKVYACEVTHQGLSSPVTKS
FNRGEC.

In some embodiments, pembrolizumab or a biosimilar thereof is administered to a patient in need, at a dose of 200 mg, once every three weeks (Q3W); or it is administered at a dose of 400 mg, once every six weeks (Q6W).

Durvalumab is a PD-L1-blocking IgG1 monoclonal antibody, and the trade name is IMFINZI.

The heavy-chain and light-chain sequences of durvalumab are as follows:

Heavy-chain amino acid sequence: (SEQ ID NO: 19)

EVQLVESGGGLVQPGGSLRLSCAASGFTFSRYWMSWVRQAPGKGLEWV
ANIKQDGSEKYYVDSVKGRFTISRDNAKNSLYLQMNSLRAEDTAVYYCAREG
GWFGELAFDYWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYICNV
NHKPSNTKVDKRVEPKSCDKTHTCPPCPAPEFEGGPSVFLFPPKPKDTLMISRT
PEVTCVVVDVSHEDPEVKFNWYVDGVEVHNAKTKPREEQYNSTYRVVSVLT
VLHQDWLNGKEYKCKVSNKALPASIEKTISKAKGQPREPQVYTLPPSREEMT
KNQVSLTCLVKGFYPSDIAVEWESNGQPENNYKTTPPVLDSDGSFFLYSKLTV
DKSRWQQGNVFSCSVMHEALHNHYTQKSLSLSPGK

Light-chain amino acid sequence: (SEQ ID NO: 20)

EIVLTQSPGTLSLSPGERATLSCRASQRVSSSYLAWYQQKPGQAPRLLIYD
ASSRATGIPDRFSGSGSGTDFTLTISRLEPEDFAVYYCQQYGSLPWTFGQGTKV
EIKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFN
RGEC

In some embodiments, durvalumab or a biosimilar thereof is administered to a patient in need weighing ≥30 kg, at a dose of 1500 mg, once every three weeks (Q3W).

In some embodiments, durvalumab or a biosimilar thereof is administered to a patient in need weighing < 30 kg, at a dose of 20 mg/kg, once every three weeks (Q3W).

In some embodiments, durvalumab or a biosimilar thereof is administered to a patient in need weighing ≥30 kg, at a dose of 1120 mg, once every three weeks (Q3W).

In some embodiments, durvalumab or a biosimilar thereof is administered to a patient in need weighing < 30 kg, at a dose of 15 mg/kg, once every three weeks (Q3W).

In another aspect, the present disclosure a use of the antibody-drug conjugate and the immune checkpoint inhibitor further in combination with a platinum drug in the preparation of a medication for treating cancer.

In optional embodiments, the platinum drug is selected from: carboplatin, cisplatin, oxaliplatin, nedaplatin (Nedaplatin), lobaplatin (lobaplatin), satraplatin (satraplatin), cycloplatin (cycloplatin), miboplatin (Miboplatin), Enloplatin, Iproplatin, and Dicycloplatin, preferably carboplatin and/or cisplatin.

In optional embodiments, the antibody-drug conjugate and the immune checkpoint inhibitor are respectively included as active ingredients in different formulations, and are administered simultaneously or not simultaneously.

In optional embodiments, the antibody-drug conjugate, the immune checkpoint inhibitor, and the platinum drug are respectively included as active ingredients in different formulations, and are administered simultaneously or not simultaneously.

In another aspect, the antibody-drug conjugate and the immune checkpoint inhibitor are included as active ingredients in a single formulation and administered.

In another aspect, the antibody-drug conjugate, the immune checkpoint inhibitor, and the platinum drug are included as active ingredients in a single formulation and administered.

In optional embodiments, the dose of the antibody-drug conjugate is 0.1 mg/kg to 12.0 mg/kg, preferably 1.0 mg/kg to 12.0 mg/kg, more preferably 4.0 mg/kg to 12.0 mg/kg, and more preferably 1.0 mg/kg, 1.2 mg/kg, 1.4 mg/kg, 1.6 mg/kg, 1.8 mg/kg, 2.0 mg/kg, 2.2 mg/kg, 2.4 mg/kg, 2.6 mg/kg, 2.8 mg/kg, 3.0 mg/kg, 3.2 mg/kg, 3.4 mg/kg, 3.6 mg/kg, 3.8 mg/kg, 4.0 mg/kg, 4.2 mg/kg, 4.4 mg/kg, 4.6 mg/kg, 4.8 mg/kg, 5.0 mg/kg, 5.2 mg/kg, 5.4 mg/kg, 5.6 mg/kg, 5.8 mg/kg, 6.0 mg/kg, 6.2 mg/kg, 6.4 mg/kg, 6.6 mg/kg, 6.8 mg/kg, 7.0 mg/kg, 7.2 mg/kg, 7.4 mg/kg, 7.6 mg/kg, 7.8 mg/kg, 8.0 mg/kg, 8.2 mg/kg, 8.4 mg/kg, 8.6 mg/kg, 8.8 mg/kg, 9.0 mg/kg, 9.2 mg/kg, 9.4 mg/kg, 9.6 mg/kg, 9.8 mg/kg, 10.0 mg/kg, 10.2 mg/kg, 10.4 mg/kg, 10.6 mg/kg, 10.8 mg/kg, 11.0 mg/kg, 11.2 mg/kg, 11.4 mg/kg, 11.6 mg/kg, 11.8 mg/kg, or 12.0 mg/kg.

In optional embodiments, the administration frequency of the antibody-drug conjugate is once every week, once every two weeks, once every three weeks, or once every four weeks.

In preferred embodiments, the antibody-drug conjugate is administered according to a starting dose of 6.0 mg/kg or 8.0 mg/kg, and the administration frequency is once every three weeks.

In optional embodiments, the dose of the immune checkpoint inhibitor is 10 mg to 2000 mg, preferably 10 mg, 15 mg, 20 mg, 25 mg, 30 mg, 40 mg, 50 mg, 60 mg, 70 mg, 75 mg, 80 mg, 90 mg, 100 mg, 110 mg, 120 mg, 125 mg, 130 mg, 140 mg, 150 mg, 155 mg, 160 mg, 165 mg, 170 mg, 175 mg, 180 mg, 185 mg, 190 mg, 195 mg, 200 mg, 205 mg, 210 mg, 215 mg, 220 mg, 225 mg, 230 mg, 235 mg, 240 mg, 245 mg, 250 mg, 255 mg, 260 mg, 265 mg, 270 mg, 275 mg, 280 mg, 285 mg, 290 mg, 295 mg, 300 mg, 310 mg, 320 mg, 325 mg, 330 mg, 340 mg, 350 mg, 360 mg, 370 mg, 375 mg, 380 mg, 390 mg, 400 mg, 450 mg, 500 mg, 550 mg, 600 mg, 650 mg, 700 mg, 750 mg, 800 mg, 850 mg, 900 mg, 950 mg, 1000 mg, 1100 mg, 1120 mg, 1150 mg, 1200 mg, 1250 mg, 1300 mg, 1350 mg, 1400 mg, 1450 mg, or 1500 mg.

In optional embodiments, the dose of the immune checkpoint inhibitor is 1.0 mg/kg to 100 mg/kg, preferably 1.0 mg/kg to 40 mg/kg, more preferably 1.0 mg/kg to 30 mg/kg, and more preferably 1.0 mg/kg, 1.2 mg/kg, 1.4 mg/kg, 1.6 mg/kg, 1.8 mg/kg, 2.0 mg/kg, 2.2 mg/kg, 2.4 mg/kg, 2.6 mg/kg, 2.8 mg/kg, 3.0 mg/kg, 3.2 mg/kg, 3.4 mg/kg, 3.6 mg/kg, 3.8 mg/kg, 4.0 mg/kg, 4.2 mg/kg, 4.4 mg/kg, 4.6 mg/kg, 4.8 mg/kg, 5.0 mg/kg, 5.2 mg/kg, 5.4 mg/kg, 5.6 mg/kg, 5.8 mg/kg, 6.0 mg/kg, 6.2 mg/kg, 6.4 mg/kg, 6.6 mg/kg, 6.8 mg/kg, 7.0 mg/kg, 7.2 mg/kg, 7.4 mg/kg, 7.6 mg/kg, 7.8 mg/kg, 8.0 mg/kg, 8.2 mg/kg, 8.4 mg/kg, 8.6 mg/kg, 8.8 mg/kg, 9.0 mg/kg, 9.2 mg/kg, 9.4 mg/kg, 9.6 mg/kg, 9.8 mg/kg, 10.0 mg/kg, 10.2 mg/kg, 10.4 mg/kg, 10.6 mg/kg, 10.8 mg/kg, 11.0 mg/kg, 11.2 mg/kg, 11.4 mg/kg, 11.6 mg/kg, 11.8 mg/kg, 12.0 mg/kg, 12.2 mg/kg, 12.4 mg/kg, 12.6 mg/kg, 12.8 mg/kg, 13.0 mg/kg, 13.2 mg/kg, 13.4 mg/kg, 13.6 mg/kg, 13.8 mg/kg, 14.0 mg/kg, 14.2 mg/kg, 14.4 mg/kg, 14.6 mg/kg, 14.8 mg/kg, 15.0 mg/kg, 15.2 mg/kg, 15.4 mg/kg, 15.6 mg/kg, 15.8 mg/kg, 16.0 mg/kg, 16.2 mg/kg, 16.4 mg/kg, 16.6 mg/kg, 16.8 mg/kg, 17.0 mg/kg, 17.2 mg/kg, 17.4 mg/kg, 17.6 mg/kg, 17.8 mg/kg, 18.0 mg/kg, 18.2 mg/kg, 18.4 mg/kg, 18.6 mg/kg, 18.8 mg/kg, 19.0 mg/kg, 19.2 mg/kg, 19.4 mg/kg, 19.6 mg/kg, 19.8 mg/kg, 20.0 mg/kg, 20.2 mg/kg, 20.4 mg/kg, 20.6 mg/kg, 20.8 mg/kg, or 30.0 mg/kg.

In optional embodiments, the administration frequency of the immune checkpoint inhibitor is once every week, once every two weeks, once every three weeks, or once every four weeks.

In preferred embodiments, the dose of the immune checkpoint inhibitor is 20 mg/kg, and the administration frequency is once every three weeks.

In optional embodiments, the administration dose of the platinum drug is calculated by area under the curve (AUC), and is 1 to 20 mg/ml/min, preferably 1 to 10 mg/ml/min, more preferably 2 mg/ml/min, 3 mg/ml/min, 4 mg/ml/min, 5 mg/ml/min, 6 mg/ml/min, 7 mg/ml/min, 8 mg/ml/min, or 9 mg/ml/min, and the administration frequency is once every week, once every two weeks, once every three weeks, or once every four weeks.

In optional embodiments, the administration dose of the platinum drug is 10 mg/m$^2$ to 500 mg/m$^2$, preferably 10 mg/m$^2$ to 200 mg/m$^2$, more preferably 25 mg/m$^2$, 50 mg/m$^2$, 75 mg/m$^2$, 100 mg/m$^2$, 125 mg/m$^2$, 150 mg/m$^2$, 175 mg/m$^2$, or 200 mg/m$^2$, and the administration frequency is once every week, once every two weeks, once every three weeks, or once every four weeks.

In optional embodiments, the platinum drug is administered for a total of 4 to 6 cycles.

In preferred embodiments, the administration dose of the platinum drug is: cisplatin 75 mg/m$^2$ or carboplatin AUC 5 mg/ml/min by intravenous infusion, and the administration frequency is once every three weeks.

In optional embodiments, the cancer is selected from at least one of the following: lung cancer, gastric cancer, liver cancer, kidney cancer, breast cancer, pancreatic cancer, prostate cancer, ovarian cancer, bladder cancer, esophageal cancer, nasopharyngeal cancer, salivary gland cancer, head and neck cancer, skin cancer, pharyngeal cancer, laryngeal cancer, gallbladder cancer, bile duct cancer, thyroid cancer, uterine cancer, vulvar cancer, penile cancer, testicular cancer, urothelial cancer, urethral cancer, colon cancer, rectal cancer, colorectal cancer, adenocarcinoma of the esophagogastric junction, gastrointestinal stromal tumor, squamous cell carcinoma, peritoneal cancer, leukemia, malignant lymphoma, plasmacytoma, myeloma, neuroepithelial tissue tumor, nerve sheath tumor, mesothelioma, Paget's disease, and sarcoma.

Further, the lung cancer is selected from non-small cell lung cancer and small cell lung cancer, the esophageal

cancer is esophageal squamous cell carcinoma, and the sarcoma is osteosarcoma.

In optional embodiments, the cancer is an advanced solid cancer that has failed first-line treatment, is intolerant to first-line treatment, or has no effective standard treatment.

In optional embodiments, the cancer is recurrent, metastatic, and/or drug-resistant cancer.

In optional embodiments, the cancer is advanced solid tumors such as small cell lung cancer, non-small cell squamous lung cancer, esophageal squamous cell carcinoma, and nasopharyngeal cancer.

In optional embodiments, the cancer is extensive-stage small cell lung cancer (SCLC) that has failed or cannot tolerate at least first-line treatment, locally advanced or metastatic non-small cell squamous lung cancer (NSCLC) that has progressed after at least first-line treatment or is intolerant thereto, unresectable, locally advanced, recurrent or metastatic esophageal squamous cell carcinoma (ESCC) that has progressed after at least first-line treatment or is intolerant thereto, locally advanced, recurrent, or metastatic nasopharyngeal cancer (NPC) that has progressed after at least first-line treatment or is intolerant thereto, or other advanced solid tumor patients who have failed adequate standard treatment or have no effective standard treatment.

In optional embodiments, the osteosarcoma is advanced osteosarcoma.

In optional embodiments, the advanced osteosarcoma is progressive osteosarcoma that has progressed after standard treatment, with a total number of previous treatment lines not exceeding 2 treatment lines.

The present disclosure further provides a pharmaceutical composition, comprising the above-described antibody-drug conjugate and immune checkpoint inhibitor, as well as one or more pharmaceutically acceptable carriers, excipients, and diluents.

In some embodiments, the pharmaceutical composition further comprises the platinum drug, as well as one or more pharmaceutically acceptable carriers, excipients, and diluents.

The present disclosure further provides a method for treating cancer, the method comprising administering in combination to a subject in need the above-described antibody-drug conjugate and immune checkpoint inhibitor, wherein the combined administration may be administration simultaneously or at different time points.

The present disclosure further provides a method for treating cancer, the method comprising administering in combination to a subject in need the above antibody-drug conjugate, immune checkpoint inhibitor, and platinum drug, wherein the combined administration may be administration simultaneously or at different time points.

In optional embodiments, the cancer is selected from at least one of the following: lung cancer, gastric cancer, liver cancer, kidney cancer, breast cancer, pancreatic cancer, prostate cancer, ovarian cancer, bladder cancer, esophageal cancer, nasopharyngeal cancer, salivary gland cancer, head and neck cancer, skin cancer, pharyngeal cancer, laryngeal cancer, gallbladder cancer, bile duct cancer, thyroid cancer, uterine cancer, vulvar cancer, penile cancer, testicular cancer, urothelial cancer, urethral cancer, colon cancer, rectal cancer, colorectal cancer, adenocarcinoma of the esophagogastric junction, gastrointestinal stromal tumor, squamous cell carcinoma, peritoneal cancer, leukemia, malignant lymphoma, plasmacytoma, myeloma, neuroepithelial tissue tumor, nerve sheath tumor, mesothelioma, Paget's disease, and sarcoma.

In optional embodiments, the lung cancer is selected from non-small cell lung cancer and small cell lung cancer, the esophageal cancer is esophageal squamous cell carcinoma, and the sarcoma is osteosarcoma.

In optional embodiments, the cancer is an advanced solid cancer that has failed first-line treatment, is intolerant thereto, or has no effective standard treatment.

In optional embodiments, the cancer is recurrent, metastatic, and/or drug-resistant cancer.

In optional embodiments, the cancer is advanced solid tumors such as small cell lung cancer, non-small cell squamous lung cancer, esophageal squamous cell carcinoma, and nasopharyngeal cancer.

In optional embodiments, the cancer is extensive-stage small cell lung cancer (SCLC) that has failed or cannot tolerate at least first-line treatment, locally advanced or metastatic non-small cell squamous lung cancer (NSCLC) that has progressed after at least first-line treatment or is intolerant thereto, unresectable, locally advanced, recurrent or metastatic esophageal squamous cell carcinoma (ESCC) that has progressed after at least first-line treatment or is intolerant thereto, locally advanced, recurrent, or metastatic nasopharyngeal cancer (NPC) that has progressed after at least first-line treatment or is intolerant thereto, or other advanced solid tumor patients who have failed adequate standard treatment or have no effective standard treatment.

In optional embodiments, the osteosarcoma is advanced osteosarcoma.

In optional embodiments, the advanced osteosarcoma is progressive osteosarcoma that has progressed after standard treatment, with a total number of previous treatment lines not exceeding 2 treatment lines.

In another aspect, the present disclosure provides the aforesaid anti-B7H3 antibody-drug conjugate for use in treating cancer, wherein the anti-B7H3 antibody-drug conjugate is used in combination with the aforesaid anti-PD-1 antibody or antigen-binding fragment thereof.

In another aspect, the present disclosure provides the aforesaid anti-B7H3 antibody-drug conjugate for use in treating cancer, wherein the anti-B7H3 antibody-drug conjugate is used in combination with the aforesaid anti-PD-L1 antibody or antigen-binding fragment thereof.

In another aspect, the present disclosure provides the aforesaid anti-B7H3 antibody-drug conjugate for use in treating cancer, wherein the anti-B7H3 antibody-drug conjugate is used in combination with the aforesaid anti-PD-1 antibody or antigen-binding fragment thereof and the aforesaid platinum drug.

In another aspect, the present disclosure provides the aforesaid anti-B7H3 antibody-drug conjugate for use in treating cancer, wherein the anti-B7H3 antibody-drug conjugate is used in combination with the aforesaid anti-PD-L1 antibody or antigen-binding fragment thereof and the aforesaid platinum drug.

In the present disclosure, the so-called "combination" is a mode of administration, which includes various situations in which two or more drugs are administered successively or simultaneously.

Modes of administration including simultaneous administration, independent formulation and co-administration, or independent formulation and successive administration all belong to the combination administration described in the present disclosure. The so-called "simultaneously" herein means that at least one dose of the anti-PD-1/PD-L1 antibody or an antigen-binding fragment thereof and an anti-B7H3 antibody-drug conjugate are administered within a certain period of time, optionally within 3 days, within 2 days, or within 1 day, wherein the two or more substances both exhibit pharmacological effects. The so-called "successive" administration includes situations in which the anti-PD-1/PD-L1 antibody or an antigen-binding fragment thereof and the anti-B7H3 antibody-drug conjugate are administered respectively in different administration cycles. The time period may be within one administration cycle, optionally within 4 weeks, within 3 weeks, within 2 weeks, or within 1 week. Such a period includes treatments in which the anti-PD-1/PD-L1 antibody or an antigen-binding fragment thereof and the anti-B7H3 antibody-drug conjugate are administered by the same route of administration or by different routes of administration.

## Terminology

In order to more easily understand the present disclosure, certain technical and scientific terms are specifically defined below. Unless otherwise expressly defined herein, all other technical and scientific terms used herein have the meanings commonly understood by a person of ordinary skill in the art to which the present disclosure belongs.

The present disclosure incorporates the entire contents of the application WO2020063673 into the present application.

The term "antibody-drug conjugate" refers to an antibody being linked to a biologically active drug through a stable linking unit. In the present disclosure, "antibody-drug conjugate" refers to a monoclonal antibody or antibody fragment being linked to a biologically active toxic drug through a stable linking unit.

The term "antibody" refers to an immunoglobulin, which is a tetrameric structure composed of two identical heavy chains and two identical light chains connected by interchain disulfide bonds. The amino acid composition and arrangement order of the immunoglobulin heavy-chain constant region are different, and therefore their antigenicity is also different. Accordingly, immunoglobulins can be divided into five classes, also referred to as immunoglobulin isotypes, namely IgM, IgD, IgG, IgA and IgE, and their corresponding heavy chains are respectively the $\mu$ chain, $\delta$ chain, $\gamma$ chain, $\alpha$ chain, and $\varepsilon$ chain. The same class of Ig can further be divided into different subclasses according to differences in the amino acid composition of its hinge region and the number and position of heavy-chain disulfide bonds, for example, IgG can be divided into IgG1, IgG2, IgG3, and IgG4. Light chains are divided, according to differences in the constant region, into $\kappa$ chains or $\lambda$ chains. In each of the five classes of Ig, the Ig may have either a $\kappa$ chain or a $\lambda$ chain.

The sequences of about 110 amino acids near the N-terminus of the antibody heavy chain and light chain vary greatly and are variable regions (the Fv region); the sequences of the remaining amino acids near the C terminus are relatively stable and are constant regions. The variable region comprises 3 hypervariable regions (HVR) and 4 framework regions with relatively conserved sequences (FR). The 3 hypervariable regions determine the specificity of the antibody, and are also called complementarity-determining regions (CDR). Each light-chain variable region (LCVR) and heavy-chain variable region (HCVR) is composed of 3 CDR regions and 4 FR regions, and the order of arrangement from the amino terminus to the carboxyl terminus is: FR1, CDR1, FR2, CDR2, FR3, CDR3, and FR4. The 3 CDR regions of the light chain refer to LCDR1, LCDR2, and LCDR3; the 3 CDR regions of the heavy chain refer to HCDR1, HCDR2, and HCDR3.

In the present disclosure, the amino acid sequences of the above-mentioned CDRs are all shown according to the Kabat definition rules. However, it is well known to those skilled in the art that in this field the CDRs of an antibody may be defined by a variety of methods, for example, the definition by Chothia based on the three-dimensional structure of the antibody and the topology of the CDR loops (Chothia et al. (1989) Nature 342:877-883, Al-Lazikani et al., "Standard conformations for the canonical structures of immunoglobulins", Journal of Molecular Biology, 273, 927-948 (1997)), by Kabat based on antibody sequence variability (Kabat et al., Sequences of Proteins of Immunological Interest, 4th Edition, U.S. Department of Health and Human Services, National Institutes of Health (1987)), by AbM (University of Bath), by Contact (University College London), by the international ImMunoGeneTics database (IMGT) (World Wide Web imgt.cines.fr/), and by the North CDR based on affinity propagation clustering using a large number of crystal structures. Those skilled in the art should understand that, unless otherwise specified, the terms "CDR" and "complementarity-determining region" of a given antibody or region thereof (for example, a variable region) should be understood as covering

complementarity-determining regions as defined by any of the above known schemes described in the present invention. Although the claimed protection scope of the present invention is based on the sequences shown according to the Kabat definition rules, the amino acid sequences corresponding to other CDR definition rules should also fall within the protection scope of the present invention.

The term "antigen-binding fragment" refers to one or more fragments of an antibody that retain the ability to specifically bind an antigen. It has been shown that a fragment of a full-length antibody may be used to perform the antigen-binding function of the antibody. Examples of binding fragments contained in "antigen-binding fragments" include (i) a Fab fragment, a monovalent fragment composed of VL, VH, CL, and CH1 domains; (ii) an $F(ab')_2$ fragment, a bivalent fragment containing two Fab fragments linked by a disulfide bridge on the hinge region; (iii) an Fd fragment composed of VH and CH1 domains; (iv) an Fv fragment composed of the VH and VL domains of a single arm of an antibody; (v) a single-domain or dAb fragment (Ward et al., (1989) Nature 341: 544-546), which is composed of a VH domain; and (vi) an isolated complementarity-determining region (CDR), or (vii) a combination of two or more isolated CDRs optionally linked by a synthetic linker.

The term "drug load" refers to the average number of cytotoxic drugs loaded on each ligand in a Formula (I) molecule, and may also be expressed as the ratio of the drug amount to the antibody amount, and the range of drug load may be 0-12, preferably 1-10 of cytotoxic drugs (D) linked to each antibody (Pc). In embodiments of the present disclosure, the drug load is expressed as n, and may also be referred to as the DAR value, exemplarily the mean values of 1, 2, 3, 4, 5, 6, 7, 8, 9, and 10. The average drug amount per ADC molecule after the conjugation reaction may be determined by conventional methods such as UV/visible spectroscopy, mass spectrometry, ELISA assays, and HPLC.

The term "immune checkpoint inhibitor" refers to an agent that inhibits the immunosuppressive system so as to activate tumor immunity.

The term "anti-PD-1 antibody or antigen-binding fragment thereof" refers to an antibody that specifically binds PD-1 (programmed cell death-1; CD279; PDCD1) and has the activity of reducing, inhibiting, and/or interfering with signal transduction caused by the interaction between PD-1 and PD-L1 or PD-L2 as a binding counterpart. The anti-PD-1 antibody used in the present disclosure is not particularly limited, as long as its clinical efficacy and safety have been demonstrated.

The term "anti-PD-L1 antibody or antigen-binding fragment thereof" refers to an antibody that specifically binds PD-L1 (programmed cell death ligand 1; CD274; B7-H1) and has the activity of reducing, inhibiting, and/or interfering with signal transduction caused by the interaction between PD-L1 and PD-1 or B7.1 (CD80) as a binding counterpart. The anti-PD-L1 antibody used in the present disclosure is not particularly limited, as long as its clinical efficacy and safety have been demonstrated.

The term "pharmaceutical composition" means a product comprising one or more active ingredients (for example, an antibody, ADC) in an optionally specific amount, as well as any product directly or indirectly produced by combining one or more active ingredients in an optionally specific amount. The different active ingredients in the pharmaceutical composition may each be independently administered in separate formulation forms, including combined synergistic enhancement by administration simultaneously or at different time points. In the present disclosure, "pharmaceutical composition" and "formulation" are not mutually exclusive.

The term "treatment" means administering to a patient an internal or external therapeutic agent, for example, a composition comprising any binding compound of the present disclosure, wherein the patient has one or more disease symptoms, and the therapeutic agent is known to have a therapeutic effect on these symptoms. Usually, the therapeutic agent is administered to the treated patient or population in an amount effective to alleviate one or more disease symptoms, so as to induce regression of such symptoms or inhibit the development of such symptoms to any clinically measurable degree. The amount of a therapeutic agent effective to alleviate any particular disease symptom (also referred to as a "therapeutically effective amount") may vary according to various factors, such as the disease state, age, and weight of the patient, as well as the ability of the drug to produce the required efficacy in the patient. Whether a disease symptom has been alleviated may be evaluated by any clinical testing method usually used by a physician or other healthcare professional to assess the severity or progression of the symptom. Although the embodiments of the present disclosure (for example, treatment methods or products) may be ineffective in alleviating each target disease symptom, it should be determined, according to any statistical test method known in the art such as Student's t-test, chi-square test, the U test according to Mann and Whitney, Kruskal-Wallis test (H test), Jonckheere-Terpstra test, and Wilcoxon test, that they should alleviate the target disease symptoms in a statistically significant number of patients.

## Description of the Drawings

Figure 1 Efficacy of drug A in combination with drug B on a subcutaneous xenograft tumor model of the human lung cancer cell line Calu-6.

Figure 2 Efficacy of drug A in combination with drug C on a subcutaneous xenograft tumor model of the human lung cancer cell line Calu-6.

Figure 3 Efficacy of drug A in combination with drug B on a subcutaneous xenograft tumor model of the human osteosarcoma cell line SJSA-1.

Figure 4 Efficacy of drug A in combination with drug C on a subcutaneous xenograft tumor model of the human osteosarcoma cell line SJSA-1.

Figure 5 Changes in body weight of mice in each group in Example 4.

**Specific Embodiments**

The present application will be explained in more detail below in conjunction with examples, and the examples of the present application are only used to illustrate the technical solution of the present application, and do not limit the substance and scope of the present application.

**Example 1.** Preparation of an anti-B7H3 antibody-drug conjugate

According to the production method described in WO2020063673, h1702DS (anti-B7H3 antibody) and an exatecan analogue were used to prepare the anti-B7H3 antibody-drug conjugate shown in the following structure, and the average value calculated by the HIC method was: n = 4.1, namely FADC-2. Wherein the heavy-chain sequence of h1702DS is as shown in SEQ ID NO: 09, and the light-chain sequence is as shown in SEQ ID NO: 10.

h1702DS

n

FADC-2

**Example 2.** Pharmacodynamic effects of single/combined administration on a PBMC-humanized NCG mouse allograft model of the human lung cancer cell line Calu-6.

**1. Experimental Materials**

**1.1 Information on the human lung cancer cell Calu-6 CDX model**

Calu-6 cells were cultured in DMEM culture medium containing 10% fetal bovine serum and 1% penicillin-streptomycin double antibiotics, in an incubator at 37°C with 5% CO2. After the cells had grown to confluence, they were passaged into flasks, and Calu-6 cells in the logarithmic growth phase were collected, counted, and inoculated.

**1.2 PBMC information**

PBMCs were purchased from Zhejiang Free Trade Zone Maishun Biotechnology Co., Ltd., batch P121090306C.

**1.3 Experimental animals**

NCG mice, female, body weight 17-19 g, were purchased from Jiangsu Jicui Yaokang Biotechnology Co., Ltd.

**1.4 Test drugs**

1) Drug A: prepared by the method in WO2020063673, and normal saline was used for drug preparation.
2) Drug B: camrelizumab for injection, the sequences of the heavy chain and light chain are as shown in SEQ ID NO: 11 and SEQ ID NO: 12, respectively. Normal saline provided by Suzhou Shengdiya Biopharmaceutical Co., Ltd., batch number 202201004F was used for drug preparation.
3) Drug C: adebrelimab injection, the sequences of the heavy chain and light chain are as shown in SEQ ID NO: 13 and SEQ ID NO: 14, respectively. Normal saline provided by Suzhou Shengdiya Biopharmaceutical Co., Ltd., batch number 202304004C was used for drug preparation.

**1.5 Experimental instruments**

Table 1. Instruments used in the experiment

| Instrument | Manufacturer | Model |
|---|---|---|
| Vernier caliper | Mitutoyo, Japan | CD-6''AX |
| Electronic balance | Sartorius | BSA2202S-CW |
| CO2 incubator | Thermo | 311 |
| Cell counter | Invitrogen | Countess$^{TM}$ 3 |
| Biological safety cabinet | Suzhou Antai | BSC-1304II A2 |
| Clean bench | Suzhou Fengshi Laboratory Animal Equipment Co., Ltd. | CJ-2F |

**1.6 Experimental reagents and consumables**

Table 2. Reagents and consumables used in the experiment

| Name | Manufactur er | Catalog number | Batch number |
|---|---|---|---|
| Matrigel | Corning | 356234 | 2278002 |
| DMEM medium | Gibco | 11995073 | 2561373 |
| Fetal bovine serum (FBS) | Gibco | 10091-148 | 2556132P |
| 0.25% Trypsin-EDTA | Gibco | 25200-056 | 2458325 |
| PBS | Gibco | 10010-023 | 2436350 |
| Pen Strep (P/S) | Gibco | 15140-122 | 2441863 |
| Trypan Blue Stain | Gibco | 15250-061 | 2337341 |
| Water for injection | Gibco | A12873-01 | 2276626 |
| Normal saline | Meilunbio | MA0083-D | MA0083-D-Nov-21H |

**2. Experimental Method**

PBMC cells were taken out from liquid nitrogen, slowly shaken to thaw in a 37 °C water bath and then centrifuged, resuspended with PBS and then inoculated. At 8 days after PBMC cell inoculation, 0.1 mL of Calu-6 cell suspension (containing $5\times10^6$ cells) was subcutaneously inoculated into the right back of NCG mice. Tumor growth was observed, and random grouping was performed according to tumor volume and animal body weight, and at the time of grouping the average tumor volume was 145 $mm^3$. The day of grouping was defined as Day 0, namely PG-D0. Administration to all animals began on the day of grouping, and the remaining animals were euthanized at the end of the experiment. The specific dosing amount and dosing regimen are shown in Table 3. Tumor volume was measured, mouse body weight was measured, and data were recorded.

**Table 3. Dosing and grouping**

| Group | Compound | Dose (mg/kg) | Number of animals | Dosing volume (μl/g)[a] | Route of administration | Dosing frequency |
|---|---|---|---|---|---|---|
| 1 | Vehicle | * | 8 | 10 | *i.p.* | QW × 3w |

(continued)

| Group | Compound | Dose (mg/kg) | Number of animals | Dosing volume (μl/g)[a] | Route of administration | Dosing frequency |
|---|---|---|---|---|---|---|
| | Drug A | 1 | 8 | 10 | *i.v.* | Single dose |
| 2 3 | Drug A | 2 | 8 | 10 | *i.v.* | Single dose |
| 4 | Drug B | 3 | 8 | 10 | *i.p.* | QW × 3w |
| 5 | Drug A + Drug B | 1 + 3 | 8 | 10 + 10 | *i.v.* + *i.p.* | Single dose + QW × 3w |
| 6 | Drug A+ Drug B | 2 + 3 | 8 | 10 + 10 | *i.v.* + *i.p.* | Single dose + QW × 3w |
| 7 | Drug C | 20 | 8 | 10 | *i.p.* | QW × 3w |
| 8 | Drug A + Drug C | 2+ 20 | 8 | 10 + 10 | *i.v.* + *i.p.* | Single dose + QW × 3w |

a. Dosing volume: dosing was performed according to 10 μl/g of mouse body weight. Dosing was stopped when body weight decreased by more than 15%, and dosing was resumed when the body weight recovered to within 10%.

The experimental indicators were to examine the effect of the drugs on tumor growth, and the specific indicators were relative tumor proliferation rate T/C (%) or tumor growth inhibition rate TGI (%). Tumor volume measurement: measurement was taken twice a week using a vernier caliper, and the tumor volume calculation formula was $V = 0.5 a \times b^2$, wherein a and b represent the length diameter and width diameter of the tumor, respectively.

Calculation of TGI (%): when there was no tumor regression, TGI (%) = [1-(average tumor volume of a treatment group at the end of dosing - average tumor volume of the treatment group at grouping) / (average tumor volume of the vehicle control group at the end of treatment - average tumor volume of the vehicle control group at grouping)] × 100%. When there was tumor regression, TGI (%) = [1- (average tumor volume of a treatment group at the end of dosing - average tumor volume of the treatment group at grouping) / average tumor volume of the treatment group at grouping] × 100%.

Calculation of T/C (%): T/C (%) = average tumor volume of a treatment group at the end of dosing / average tumor volume of the vehicle control group at the end of treatment × 100%.

At the end of the experiment (PG-D21), all animals were euthanized in sequence by group by means of $CO_2$ asphyxiation. After the animals were euthanized, the tumor masses were stripped, weighed, and photographed.

All data are expressed as Mean ± SEM. Based on the tumor volume data of each group at different time points, statistical analysis was performed using Dunnett's multiple comparisons test in Two-way ANOVA to evaluate differences between groups. Dunnett's multiple comparisons test in One-way ANOVA was used to analyze differences in tumor volume between groups. A t-test was used to analyze the difference in tumor volume between two groups. GraphPad Prism 9 was used for all data analysis, and p<0.05 was considered a significant difference.

### 3. Experimental Results

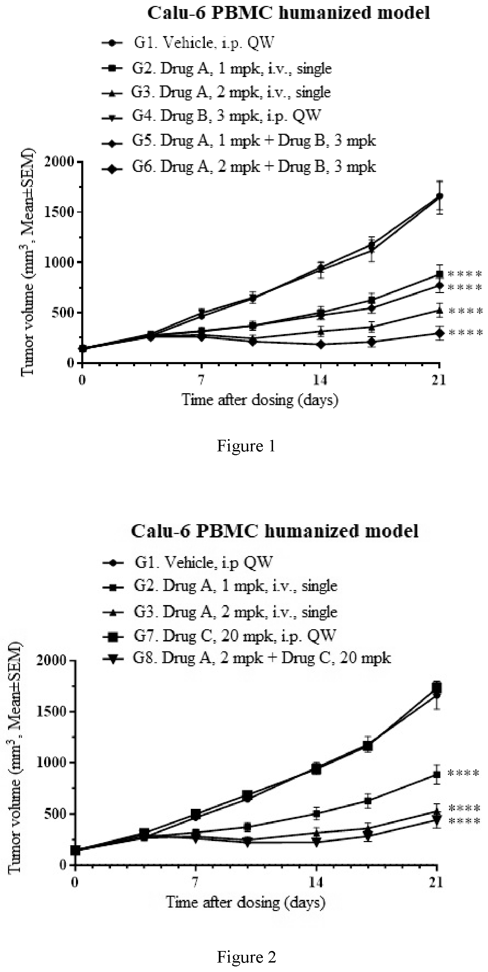

The growth inhibitory effects of drug A, drug B, and drug C alone or in combination on subcutaneous xenograft tumors of human lung cancer cells Calu-6 are shown in Table 4 and Figures 1 and 2, and in the figures, the data of the Vehicle group, drug A_1 mg/kg group, and drug A_2 mg/kg group are experimental data from the same group.

**Table 4. Evaluation of the tumor growth inhibitory efficacy of drug A, drug B, and drug C alone or in combination in the Calu-6 xenograft tumor model**

| Group | Tumor volu ume ($mm^3$) [a] | | T/C (%) | TGI (%) | *p* value[b] |
|---|---|---|---|---|---|
| | Day 0 | Day 21 | Day 21 | Day 21 | |
| Vehicle | 145±10 | 1663±136 | * | * | * |
| Drug A_1 mg/kg | 145±9 | 885±96 | 53.21 | 51.25 | < 0.0001 |

(continued)

| Group | Tumor volu ume (mm$^3$) [a] | | T/C (%) | TGI (%) | *p* value[b] |
|---|---|---|---|---|---|
| | Day 0 | Day 21 | Day 21 | Day 21 | |
| Drug A _2 mg/kg | 145±9 | 527±70 | 31.69 | 74.82 | < 0.0001 |
| Drug B _3 mg/kg | 145±9 | 1648±168 | 99.13 | 0.97 | 0.9999 |
| Drug A _1 mg/kg + Drug B_3 mg/kg | 145±9 | 773±71 | 46.47 | 58.63 | < 0.0001 |
| Drug A _2 mg/kg + Drug B _3 mg/kg | 145±9 | 298±70 | 17.89 | 89.93 | < 0.0001 |
| Drug C_20 mg/kg | 145±9 | 1732±59 | 104.16 | -4.55 | 0.9966 |
| Drug A _2 mg/kg + Drug C_20 mg/kg | 145±10 | 443±79 | 26.65 | 80.34 | < 0.0001 |

a. Mean ± SEM;
b. A statistical analysis was performed using Dunnett's multiple comparisons test in One-way ANOVA to compare with the Vehicle group. In addition, statistical analysis was performed using a t-test, and for the drug A_2 mg/kg combined with drug B_3 mg/kg group, compared respectively with the drug A_2 mg/kg group and the drug B_3 mg/kg group, the *p* values were 0.0364 and < 0.0001, respectively; for the drug A_2 mg/kg combined with drug C_20 mg/kg group, compared with the drug C_20 mg/kg group, the *p* value was < 0.0001.

In the PBMC-humanized human lung cancer cell line Calu-6 xenograft tumor model, the tumor inhibition rates of drug A_1 mg/kg and drug A_2 mg/kg monotherapy were 51.25% and 74.82%, respectively, showing significant and dose-dependent tumor inhibitory effects. The tumor inhibition rates of drug B_3 mg/kg and drug C_20 mg/kg monotherapy were 0.97% and -4.55%, respectively, not show obvious tumor inhibitory effects. The tumor inhibition rates of treatment with drug A_1 mg/kg and drug A_2 mg/kg in combination with drug B_3 mg/kg were 58.63% and 89.93%, respectively, wherein the drug A_2 mg/kg combined with drug B_3 mg/kg treatment group significantly improved the tumor inhibitory effect as compared with both the drug A_2 mg/kg monotherapy group and the drug B_3 mg/kg monotherapy group, the difference being statistically significant ($p < 0.05$). The tumor inhibition rate of treatment with drug A_2 mg/kg in combination with drug C_20 mg/kg was 80.34%, and the tumor inhibitory effect was significantly better in the combination treatment group than in the drug C_20 mg/kg monotherapy group ($p < 0.05$), and was better than in the drug A_2 mg/kg monotherapy group. In addition, the mice did not have treatment-related death or other abnormal symptoms, indicating that the tumor-bearing mice could well tolerate drug A treatment alone and in combination with drug B and drug C.

During the experiment, the average body weight of the animals in the Vehicle group was relatively stable, and at the experimental endpoint (Day 21), the average body weight change rate of the animals in this group was 8.97%. The average body weight change rates of the tumor-bearing mice in the drug A_1 mg/kg monotherapy group and the drug A_2 mg/kg monotherapy group were 8.27% and 6.22%, respectively. The average body weight change rates of the tumor-bearing mice in the drug B_3 mg/kg monotherapy group and its combination treatment groups with drug A_1 mg/kg and drug A_2 mg/kg were -3.79%, 1.73%, and -1.53%, respectively. The average body weight change rates of the tumor-bearing mice in the drug C_20 mg/kg monotherapy group and its combination treatment group with drug A_2 mg/kg were 6.58% and 1.84%, respectively. It is worth noting that GvHD caused by PBMC inoculation humanization was the main cause of the decrease in animal body weight, and in addition, the mice did not have treatment-related death or other abnormal symptoms, indicating that the tumor-bearing mice could well tolerate monotherapy with the test drug A and its combination treatment with drug B and drug C.

In summary, the tumor-bearing mice tolerated both drug A monotherapy and its combination treatment with drug B and drug C, the antitumor efficacy of the drug A_2 mg/kg combined with drug B_3 mg/kg treatment group was significantly better than that of each monotherapy treatment group, and the antitumor efficacy of the drug A_2 mg/kg combined with drug C_20 mg/kg treatment group was significantly better than that of the drug C_20 mg/kg monotherapy treatment group, and was better than that of the drug A_2 mg/kg monotherapy treatment group.

**Example 3.** Clinical study of anti-B7H3 antibody-drug conjugate in combination with an immune checkpoint inhibitor ± a platinum drug for treating advanced solid tumors

This study is used to evaluate the safety, tolerability, and efficacy of each combination treatment regimen in patients with advanced solid tumors, and the study endpoints include but are not limited to: the MTD or maximum applicable dose (MAD) of intravenous infusion administration of the anti-B7H3 antibody-drug conjugate in the combination treatment regimens, the objective response rate (ORR), disease control rate (DCR), duration of response (DoR), progression-free survival (PFS), and overall survival (OS) assessed by investigators according to the RECIST 1.1

standard, the pharmacokinetic characteristics of intravenous infusion administration of the anti-B7H3 antibody-drug conjugate in the combination treatment regimens, and the immunogenicity of the anti-B7H3 antibody-drug conjugate in the combination treatment regimens.

1. Names of test drugs:

(1) Anti-B7H3 antibody-drug conjugate

Dosage form: injection (lyophilized powder), specification: 100 mg/vial, packaged in a 20 mL borosilicate glass injection vial, manufacturer: Shanghai Hansoh Biomedical Technology Co., Ltd.

(2) Immune checkpoint inhibitor

Adebrelimab, dosage form: injection, specification: 600 mg (12 ml)/vial, packaged in a borosilicate glass injection vial, manufacturer: Suzhou Shengdiya Biopharmaceutical Co., Ltd.

(3) Platinum drugs

Carboplatin, dosage form: injection, specification: 50 mg (10 mL)/100 mg (10 mL), ampoule packaging, manufacturer: Qilu Pharmaceutical Co., Ltd.

Cisplatin, dosage form: injection, specification: 6 ml: 30 mg, packaged in a controlled antibiotic vial, manufacturer: Jiangsu Hansoh Pharmaceutical Group Co., Ltd.

2. Target population:

Patients with advanced solid tumors who have failed first-line treatment, are intolerant thereto, or have no effective standard treatment, specifically as follows: (1) extensive-stage small cell lung cancer (SCLC) that has failed at least first-line treatment or cannot tolerate it; (2) untreated extensive-stage small cell lung cancer (SCLC); (3) locally advanced or metastatic non-small cell squamous lung cancer (NSCLC) that has progressed after at least first-line treatment or is intolerant thereto; (4) locally advanced or metastatic non-small cell squamous lung cancer (NSCLC) untreated in the advanced stage; (5) unresectable locally advanced recurrent or metastatic esophageal squamous cell carcinoma (ESCC) that has progressed after at least first-line treatment or is intolerant thereto; (6) unresectable locally advanced recurrent or metastatic esophageal squamous cell carcinoma (ESCC) untreated in the advanced stage; (7) locally advanced recurrent or metastatic nasopharyngeal cancer (NPC) that has progressed after at least first-line treatment or is intolerant thereto; (8) locally advanced recurrent or metastatic nasopharyngeal cancer (NPC) untreated in the advanced stage; and (9) patients with other advanced solid tumors who have failed adequate standard treatment or have no effective standard treatment, and who are assessed by investigators as likely to benefit from the study treatment.

3. Dosing regimen:

In this study, every 3 weeks (21 days) constituted one treatment cycle (C).

Cohort 1a:

Recommended priority order of administration: adebrelimab is administered first, and after completion of its administration, anti-B7H3 antibody-drug conjugate treatment is administered after an interval of at least 30 min. The entire dosing regimen (adebrelimab + anti-B7H3 antibody-drug conjugate) is required to be completed within 72 h, calculated from the start of administration of the first drug of the combination therapy until the completion of administration of the last drug.

Adebrelimab: 20 mg/kg. The total administered amount is calculated according to the subject's body weight measurement result before administration, and it is administered by intravenous infusion, Q3W, until objective disease progression (excluding drug donation) or reaching other criteria for termination of study treatment specified in the protocol. Anti-B7H3 antibody-drug conjugate: administered according to a starting dose of 8.0 mg/kg and by intravenous infusion, Q3W, until objective disease progression (excluding drug donation) or reaching other criteria for termination of study treatment specified in the protocol.

Cohort 1b:

Recommended priority order of administration: adebrelimab is administered first, and after completion of administration, anti-B7H3 antibody-drug conjugate treatment is administered after an interval of at least 30 min; after completion of administration of the anti-B7H3 antibody-drug conjugate, cisplatin/carboplatin treatment is administered after an interval of at least 60 min. The entire dosing regimen (adebrelimab + anti-B7H3 antibody-drug conjugate + cisplatin/carboplatin) is required to be completed within 72 h, calculated from the start of administration of the first drug of the combination therapy until the completion of administration of the last drug.

Adebrelimab: 20 mg/kg. The total administered amount is calculated according to the subject's body weight measurement result before administration, and it is administered by intravenous infusion, Q3W, until objective disease progression (excluding drug donation) or reaching other criteria for termination of study treatment specified in the protocol. Anti-B7H3 antibody-drug conjugate: administered according to a starting dose of 6.0 mg/kg and by intravenous infusion, Q3W, until objective disease progression (excluding drug donation) or reaching other criteria for termination of study treatment specified in the protocol.

Cisplatin/carboplatin: cisplatin 75 mg/m2 or carboplatin AUC 5 mg/ml/min by intravenous infusion, Q3W, administered for a total of 4-6 cycles. The investigator may select cisplatin or carboplatin according to the specific condition of the subject. During treatment, interchange between cisplatin and carboplatin is allowed because of tolerability issues, but the subject should be fully informed of the risk of cross-allergy. If the subject cannot tolerate the drug because of safety, treatment with that drug is terminated.

**Example 4. Evaluation of the in vivo inhibitory effect of drug A combined with drug B or drug C on transplanted human osteosarcoma tumors in mice 1. Experimental Materials**

The human osteosarcoma cell line SJSA-1 was purchased from Guangzhou Jennio Biotech Co., Ltd., monolayer-cultured in vitro, and the culture conditions were culture with DMEM medium plus 10% fetal bovine serum in a cell incubator at 37°C with 5% $CO_2$. Routine digestion treatment and passaging were performed twice a week with trypsin-EDTA. When the cell confluence was 80%-90% and the quantity reached the requirement, the cells were collected, counted, and inoculated.

NOG-dKO mice, female, body weight 16-22 g, were purchased from Zhejiang Vital River Laboratory Animal Technology Co., Ltd.

**2. Experimental Method**

PBMC cells (human peripheral blood mononuclear cells) were resuspended in PBS, adjusted to a density of $5.0\times10^7$ cells/mL, injected through the tail vein, with each mouse being inoculated with 0.1 mL ($5.0\times10^6$ cells/mouse);

One week after inoculation of the PBMC cells, the SJSA-1 cells were resuspended in PBS, adjusted to a density of $1.0\times10^8$ cells/mL, the resuspended cells were mixed with an equal volume of Matrigel, and inoculated subcutaneously into the right back of each mouse, with each mouse being inoculated with 0.1 mL ($5.0\times10^6$ cells/mouse);

When the average tumor volume grew to 50-100 $mm^3$, grouping was performed (D0), and drug administration was performed (D0). The mice were administered by tail vein injection (i.v., drug A) or by intraperitoneal injection (i.p., drug B or drug C), as a single dose (Single dose, drug A) or twice weekly (BIW, drug B or drug C); the dosing volume was 10 mL/kg; the vehicle group was given the same volume of "vehicle" (Saline); the specific dosing amounts and dosing regimens are shown in Table 1. Tumor volume was measured, mouse body weight was weighed, and data were recorded.

The experimental indicators were to examine the effect of the drugs on tumor growth, and the specific indicators were T/C% or tumor growth inhibition rate TGI (%).

Tumor diameter was measured with a vernier caliper, and the calculation formula for tumor volume (V) was: $V = 1/2\times a\times b^2$, wherein a and b represent the long diameter and short diameter of the tumor, respectively.

T/C (%) = $(T-T_i)/(C-C_i)\times100$, wherein T and C are the tumor volumes of the dosing group and the control group at the end of the experiment, and $T_i$ and $C_i$ are the tumor volumes of the dosing group and the control group at the beginning of the experiment.

$$\text{Tumor growth inhibition rate (TGI) (\%)} = 100\text{-T/C (\%)}.$$

When tumor regression occurs, tumor growth inhibition rate (TGI) (%) = 100-(T-Ti)/Ti $\times$100.

If the tumor shrinks relative to the initial volume, that is, when $T < T_i$ or $C < C_i$, it is defined as partial regression of the tumor (PR); if the tumor completely disappears, it is defined as complete regression of the tumor (CR).

At the end of the experiment, upon reaching the experimental endpoint, or when the average tumor volume

reached 2,000 $mm^3$, the mice were euthanized, followed by dissection to collect the tumors and photograph them.

The experimental data were analyzed and plotted using GraphPad Prism 9. Based on the tumor volume data of each group at different time points, statistical analysis was performed using Two-way ANOVA to evaluate differences between groups. $P < 0.05$ was defined as a statistically significant difference.

**Table 5. Dosing regimens of drug A in combination with B or drug C in the human osteosarcoma SJSA-1 model**

| Grou p | Grouping | Dose mg/kg | Dosing volume mL/kg | Dosing cycle | Route of administr ation |
|---|---|---|---|---|---|
| 1 | Vehicle | * | 10 | Single dose + BIW*3 weeks | *i.v.+ i.p.* |
| 2 | Drug A | 12 | 10 | Single dose | *i.v.* |
| 4 | Drug B | 10 | 10 | BIW*3 weeks | *i.p.* |
| 5 | Drug C | 30 | 10 | BIW*3 weeks | *i.p.* |
| 6 | Drug A + drug B | 12+10 | 10+10 | Single dose + BIW*3 weeks | *i.v.+ i.p.* |
| 7 | Drug A + drug C | 12+30 | 10+10 | Single dose + BIW*3 weeks | *i.v. + i.p.* |

**3. Experimental Results**

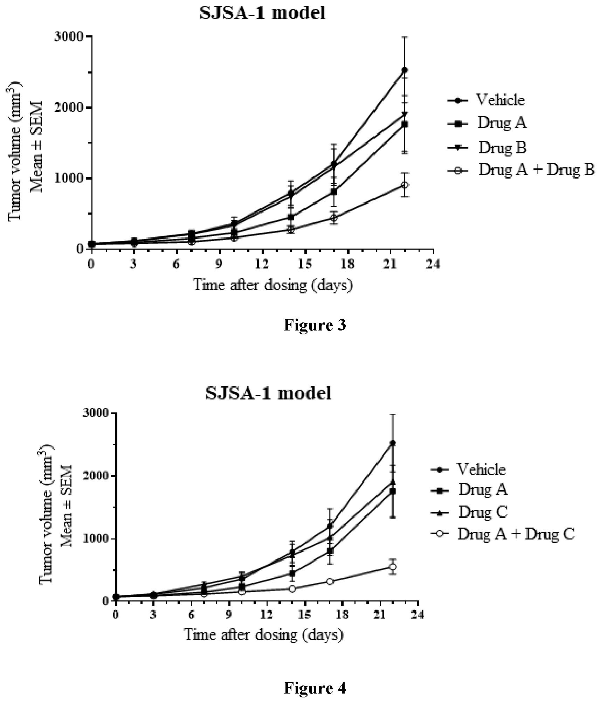

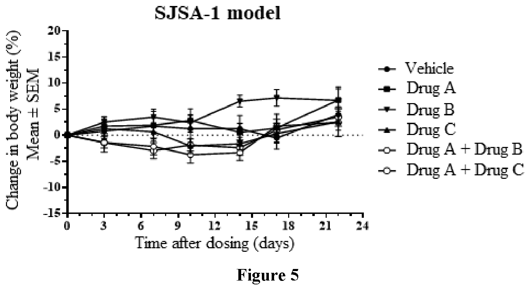

The growth inhibitory effect of drug A in combination with drug B on the SJSA-1 model is shown in Table 6 and Figure 3, and the body weight changes of mice in each group are shown in Figure 5; the growth inhibitory effect of drug A in combination with drug C on the SJSA-1 model is shown in Table 7 and Figure 4, and the body weight changes of mice in each group are shown in Figure 5.

**Table 6. Growth inhibitory effect of drug A in combination with drug B on the SJSA-1 model**

| NO. | Group | Average tumor volume ($mm^3$) ± SEM | | TGI(%) | P value |
|---|---|---|---|---|---|
| | | Day 0 | Day 22 | Day 22 | Day 22 |
| G1 | Vehicle | 73±4 | 2,531±462 | * | * |
| G2 | Drug A | 73±5 | 1,763±412 | 31.26 | 0.0040## |
| G3 | Drug B | 73±4 | 1,900±521 | 25.67 | 0.0176### |
| G5 | Drug A + drug B | 73±4 | 907±168 | 66.08 | <0.001 |

Note: *P* value Day 22: values obtained by analysis based on the tumor volume of each animal in different groups using the vehicle group as the control;
#: compared with the drug A + drug B combination group, analysis was performed using Two-way ANOVA, $^{\#\#}P < 0.01$, $^{\#\#\#}P < 0.001$.

At the end of the experiment (Day 22), the average tumor volumes of the drug A, drug B, and drug A + drug B groups were 1,763 $mm^3$, 1,900 $mm^3$, and 907 $mm^3$, respectively, and the tumor inhibition rates were 31.26%, 25.67%, and 66.08%, respectively; compared with the vehicle group, all dosing groups had significant tumor-inhibitory effects (*P* values were 0.0040, 0.0176, and < 0.001, respectively); the drug A + drug B combination treatment group (G5) showed statistically significant differences compared with the respective monotherapy groups (G2 and G3) ($P < 0.01$ and $P < 0.001$), exhibiting a relatively strong tumor-inhibitory effect.

**Table 7. Growth-inhibitory effect of drug A in combination with drug C on the** SJSA-1 **model**

| NO. | Group | Average tumor volume ($mm^3$) ± SEM | | TGI(%) | P value |
|---|---|---|---|---|---|
| | | Day 0 | Day 22 | Day 22 | Day 22 |
| G1 | Vehicle | 73±4 | 2,531±462 | * | * |
| G2 | Drug A | 73±5 | 1,763±412 | 31.26 | 0.0051### |
| G4 | Drug C | 73±4 | 1,911±575 | 25.25 | 0.0234### |

(continued)

| NO. | Group | Average tumor volume ($mm^3$) ± SEM | | TGI(%) | P value |
|---|---|---|---|---|---|
| | | Day 0 | Day 22 | Day 22 | Day 22 |
| G6 | Drug A + drug C | 73±5 | 558±118 | 80.26 | <0.001 |

Note: $P$ value Day 22: values obtained by analysis based on the tumor volume of each animal in different groups using the vehicle group as the control;
$^{\#}$: compared with the drug A + drug C combination group, analysis was performed using Two-way ANOVA, $^{\#\#\#}P < 0.001$.

At the end of the experiment (Day 22), the average tumor volumes of the drug A, drug C, and drug A + drug C groups were 1,763 $mm^3$, 1,911 $mm^3$, and 558 $mm^3$, respectively, and the tumor inhibition rates were 31.26%, 25.25%, and 80.26%, respectively; compared with the vehicle group, all dosing groups had significant tumor-inhibitory effects ($P$ values were 0.0051, 0.0234, and < 0.001, respectively); the drug A + drug C combination treatment group (G6) showed statistically significant differences compared with the respective monotherapy groups (G2 and G4) ($P < 0.001$), exhibiting a relatively strong tumor-inhibitory effect.

Throughout the entire experiment, the SJSA-1 tumor-bearing mice had relatively good tolerability to drug A, drug B, drug C, as well as drug A combined with drug B and drug A combined with drug C.

Overall, in the human osteosarcoma cell SJSA-1 mouse model, treatment with combined drug A + drug B and treatment with combined drug A + drug C had better tumor-inhibitory effects than the respective monotherapies.

**Example 5.** Clinical study of anti-B7H3 antibody-drug conjugate in combination with an immune checkpoint inhibitor for treating advanced osteosarcoma

To evaluate the safety, tolerability, pharmacokinetics (PK), and efficacy of the anti-B7H3 antibody-drug conjugate for injection combination treatment regimen in subjects with advanced osteosarcoma.

An expansion study in the target population will be conducted at a dose determined to be safe and potentially effective, to obtain further safety, PK, and efficacy of the combination treatment regimen, and to determine the target dose of the combination treatment regimen.

1. Names of test drugs:

(1) Anti-B7H3 antibody-drug conjugate

Dosage form: injection (lyophilized powder), specification: 100 mg/vial, packaged in a 20 mL borosilicate glass injection vial, manufacturer: Shanghai Hansoh Biomedical Technology Co., Ltd.

(2) Immune checkpoint inhibitor

Adebrelimab, dosage form: injection, specification: 600 mg (12 ml)/vial, packaged in a borosilicate glass injection vial, manufacturer: Suzhou Shengdiya Biopharmaceutical Co., Ltd.

2. Target population:

Cohort 2

Dose-escalation/expansion population: progressive osteosarcoma that has progressed after standard treatment and also meets the following conditions:

① Definition of progressive osteosarcoma: a. the primary tumor or locally recurrent tumor cannot be radically cured by surgery or other local treatment means (for example: stereotactic radiosurgery treatment, argon-helium knife, focused ultrasound knife, etc.), or the patient refuses surgery or other local treatment. b. distant metastasis has already occurred, and the metastatic tumor cannot be radically cured by surgery or other local treatment means (for example: stereotactic radiosurgery treatment, argon-helium knife, focused ultrasound knife, etc.), or the patient refuses surgery or other local treatment.
② Progression after standard treatment, wherein standard treatment is defined as receiving 2 or more of the following standard therapeutic drugs: methotrexate, anthracyclines, cisplatin, or ifosfamide. If the subject has previously received only first-line treatment (≥ 2 standard therapeutic drugs) and disease progression occurs within 6 months

after the end of treatment, the subject may be included in the study; for patients in whom the disease progression occurs after 6 months, the investigator needs to determine whether the subject is included in the treatment study through risk-benefit assessment.

③ The total number of previous treatment lines does not exceed 2 treatment lines.

Note:
For subjects that are enrolled because of intolerance to the last treatment, the investigator needs to record the reason for intolerance to the current treatment or provide an explanatory statement.

When the cumulative dose of anthracyclines reaches a specific dose, it may be counted as intolerable, and the maximum lifetime cumulative dose of commonly used anthracyclines is shown in Appendix 13.

If a subject in the dose-escalation period refuses to receive first-line standard treatment, enrollment is allowed after the investigator and the sponsor communicate and reach an agreement and after approval by the ethics committee.

3. Dosing regimen

For all two-drug combination cohorts (Cohort 2a), the preset combination starting dose of the anti-B7H3 antibody-drug conjugate is 10.0 mg/kg Q3W, and in Cohort 2a, the dose of the combination drug is fixed, and only the dose of the anti-B7H3 antibody-drug conjugate is adjusted.

Recommended priority order of administration: adebrelimab is administered first, and after completion of its administration, anti-B7H3 antibody-drug conjugate treatment is administered after an interval of at least 30 min. The entire dosing regimen (adebrelimab + anti-B7H3 antibody-drug conjugate) is required to be completed within 72 h, calculated from the start of administration of the first drug of the combination therapy until the completion of administration of the last drug.

Adebrelimab: 20 mg/kg by intravenous infusion, Q3W, until objective disease progression (excluding continued treatment after disease progression) or reaching other criteria for termination of study treatment specified in the protocol.

Anti-B7H3 antibody-drug conjugate: administered according to the preset dose of 10 mg/kg by intravenous infusion, Q3W, until objective disease progression (excluding continued treatment after disease progression) or reaching other criteria for termination of study treatment specified in the protocol.

Although the specific embodiments of the present invention have been described in detail, those skilled in the art will understand that various modifications and changes may be made to the details according to all teachings already published, and these changes are all within the protection scope of the present invention. The entirety of the present invention is defined by the appended claims and any equivalents thereof.

## Claims

**1.** Use of an antibody-drug conjugate and an immune checkpoint inhibitor in combination in the preparation of a medication for treating cancer, wherein a structure of the antibody-drug conjugate is as shown in Formula (I):

(I)

in the formula:

n is 1 to 10, preferably 2 to 8, more preferably 3 to 8, and n is a decimal or an integer;
Pc is an anti-B7H3 antibody or an antigen-binding fragment thereof.

**2.** The use according to claim 1, wherein the anti-B7H3 antibody or antigen-binding fragment thereof comprises: heavy-chains HCDR1, HCDR2, and HCDR3 as shown by the amino acid sequences of SEQ ID NO: 01, 02, and 03,

respectively, and light-chains LCDR1, LCDR2, and LCDR3 as shown by the amino acid sequences of SEQ ID NO: 04, 05, and 06, respectively.

**3.** The use according to claim 1 or 2, wherein the anti-B7H3 antibody or an antigen-binding fragment thereof is selected from a humanized antibody or a fragment thereof.

**4.** The use according to claim 3, wherein the anti-B7H3 antibody or an antigen-binding fragment thereof comprises a heavy-chain constant region of the human IgG1, IgG2, IgG3, or IgG4 isotype, and comprises a light-chain constant region of $\kappa$ or $\lambda$; preferably, the anti-B7H3 antibody or an antigen-binding fragment thereof comprises a heavy-chain constant region of the IgG1 or IgG4 isotype.

**5.** The use according to claim 3, wherein the heavy-chain variable region sequence of the anti-B7H3 antibody or an antigen-binding fragment thereof is the sequence as shown in SEQ ID NO: 07 or a variant thereof, and the light-chain variable region sequence is the sequence as shown in SEQ ID NO: 08 or a variant thereof.

**6.** The use according to any one of claims 1 to 5, wherein the heavy-chain sequence of the anti-B7H3 antibody or an antigen-binding fragment thereof is the sequence as shown in SEQ ID NO: 09 or a variant thereof, and the light-chain sequence is the sequence as shown in SEQ ID NO: 10 or a variant thereof.

**7.** The use according to any one of claims 1 to 6, wherein the immune checkpoint inhibitor is selected from antibodies targeting PD-1, PD-L1, CTLA-4, LAG-3, TIM-3, TIGIT, BTLA, A2aR, B7-H3, and B7-H4, or an antigen-binding fragment thereof, preferably antibodies targeting PD-1 and PD-L1, or antigen-binding fragments thereof.

**8.** The use according to any one of claims 1 to 7, wherein the immune checkpoint inhibitor is selected from toripalimab (Toripalimab), sintilimab (Sintilimab), camrelizumab (Camrelizumab), dostarlimab (Dostarlimab), tislelizumab (Tislelizumab), zimberelimab (Zimberelimab), penpulimab (Penpulimab), serplulimab (Serplulimab), pucotenlimab (Pucotenlimab), pembrolizumab (Pembrolizumab), nivolumab (Nivolumab), sugemalimab (Sugemalimab), envafolimab (Envafolimab), adebrelimab (Adebrelimab), atezolizumab (Atezolizumab), durvalumab (Durvalumab), ipilimumab (Ipilimumab), and candonilimab antibody (Candonilimab), preferably camrelizumab (Camrelizumab), adebrelimab (Adebrelimab), dostarlimab (Dostarlimab), pembrolizumab (Pembrolizumab), and durvalumab (Durvalumab).

**9.** The use according to any one of claims 1 to 8, further in combination with a platinum drug.

**10.** The use according to claim 9, wherein the platinum drug is selected from: carboplatin, cisplatin, oxaliplatin, nedaplatin (Nedaplatin), lobaplatin (lobaplatin), satraplatin (satraplatin), cycloplatin (cycloplatin), miboplatin (Miboplatin), Enloplatin, Iproplatin, and Dicycloplatin, preferably carboplatin and/or cisplatin.

**11.** The use according to any one of claims 1 to 10, wherein the antibody-drug conjugate and the immune checkpoint inhibitor are respectively included as active ingredients in different formulations, and are administered simultaneously or not simultaneously.

**12.** The use according to any one of claims 1 to 10, wherein the antibody-drug conjugate and the immune checkpoint inhibitor are included as active ingredients in a single formulation and administered.

**13.** The use according to claim 9 or 10, wherein the administration dose of the platinum drug is calculated by area under the curve (AUC), and is 1 to 10 mg/ml/min, preferably 3 mg/ml/min, 4 mg/ml/min, 5 mg/ml/min, 6 mg/ml/min, 7 mg/ml/min, or 8 mg/ml/min, and the administration frequency is once every week, once every two weeks, once every three weeks, or once every four weeks.

**14.** The use according to claim 9 or 10, wherein the administration dose of the platinum drug is 10 mg/$m^2$ to 500 mg/$m^2$, preferably 10 mg/$m^2$ to 200 mg/$m^2$, more preferably 25 mg/$m^2$, 50 mg/$m^2$, 75 mg/$m^2$, 100 mg/$m^2$, 125 mg/$m^2$, 150 mg/$m^2$, 175 mg/$m^2$, or 200 mg/$m^2$, and the administration frequency is once every week, once every two weeks, once every three weeks, or once every four weeks.

**15.** The use according to any one of claims 1 to 14, wherein the dose of the antibody-drug conjugate is 0.1 mg/kg to 12.0 mg/kg, preferably 1.0 mg/kg to 12.0 mg/kg, and the administration frequency is once every week, once every two weeks, once every three weeks, or once every four weeks.

16. The use according to any one of claims 1 to 15, wherein the dose of the immune checkpoint inhibitor is 10 mg to 2000 mg, preferably 50 mg, 100 mg, 150 mg, 200 mg, 250 mg, 300 mg, 400 mg, 500 mg, 600 mg, 700 mg, 800 mg, 900 mg, 1000 mg, 1100 mg, 1120 mg, 1200 mg, 1300 mg, 1400 mg, or 1500 mg, and the administration frequency is once every week, once every two weeks, once every three weeks, or once every four weeks.

17. The use according to any one of claims 1 to 16, wherein the dose of the immune checkpoint inhibitor is 1.0 mg/kg to 100 mg/kg, preferably 1.0 mg/kg to 40 mg/kg, more preferably 1.0 mg/kg to 30 mg/kg, and the administration frequency is once every week, once every two weeks, once every three weeks, or once every four weeks.

18. The use according to any one of claims 1 to 17, wherein the cancer is selected from at least one of the following: lung cancer, gastric cancer, liver cancer, kidney cancer, breast cancer, pancreatic cancer, prostate cancer, ovarian cancer, bladder cancer, esophageal cancer, nasopharyngeal cancer, salivary gland cancer, head and neck cancer, skin cancer, pharyngeal cancer, laryngeal cancer, gallbladder cancer, bile duct cancer, thyroid cancer, uterine cancer, vulvar cancer, penile cancer, testicular cancer, urothelial cancer, urethral cancer, colon cancer, rectal cancer, colorectal cancer, adenocarcinoma of the esophagogastric junction, gastrointestinal stromal tumor, squamous cell carcinoma, peritoneal cancer, leukemia, malignant lymphoma, plasmacytoma, myeloma, neuroepithelial tissue tumor, nerve sheath tumor, mesothelioma, Paget's disease, and sarcoma.

19. The use according to claim 18, wherein the lung cancer is selected from non-small cell lung cancer and small cell lung cancer, the esophageal cancer is esophageal squamous cell carcinoma, and the sarcoma is osteosarcoma.

20. The use according to any one of claims 1 to 17, wherein the cancer is an advanced solid cancer that has failed first-line treatment, is intolerant thereto, or has no effective standard treatment.

21. The method according to claim 19, wherein the osteosarcoma is advanced osteosarcoma, preferably progressive osteosarcoma, which has progressed after standard treatment, and has previously received no more than 2 treatment lines in total.

22. A pharmaceutical composition, comprising the antibody-drug conjugate and immune checkpoint inhibitor as defined in any one of claims 1 to 21, and one or more pharmaceutically acceptable carriers, excipients, and diluents.

23. A method for treating cancer, the method comprising combined administration to a subject in need the antibody-drug conjugate as defined in any one of claims 1 to 21, the immune checkpoint inhibitor as defined in any one of claims 1 to 21, and optionally the platinum drug as defined in any one of claims 9-20, wherein the combined administration may be administration simultaneously or at different time points.

24. The method according to claim 23, wherein the cancer is selected from at least one of the following: lung cancer, gastric cancer, liver cancer, kidney cancer, breast cancer, pancreatic cancer, prostate cancer, ovarian cancer, bladder cancer, esophageal cancer, nasopharyngeal cancer, salivary gland cancer, head and neck cancer, skin cancer, pharyngeal cancer, laryngeal cancer, gallbladder cancer, bile duct cancer, thyroid cancer, uterine cancer, vulvar cancer, penile cancer, testicular cancer, urothelial cancer, urethral cancer, colon cancer, rectal cancer, colorectal cancer, adenocarcinoma of the esophagogastric junction, gastrointestinal stromal tumor, squamous cell carcinoma, peritoneal cancer, leukemia, malignant lymphoma, plasmacytoma, myeloma, neuroepithelial tissue tumor, nerve sheath tumor, mesothelioma, Paget's disease, and sarcoma.

25. The method according to claim 24, wherein the lung cancer is selected from non-small cell lung cancer and small cell lung cancer, the esophageal cancer is esophageal squamous cell carcinoma, and the sarcoma is osteosarcoma.

26. The method according to claim 23, wherein the cancer is an advanced solid cancer that has failed first-line treatment, is intolerant thereto, or has no effective standard treatment.

27. The method according to claim 25, wherein the osteosarcoma is advanced osteosarcoma, preferably progressive osteosarcoma, which has progressed after standard treatment, and has previously received no more than 2 treatment lines in total.

Calu-6 PBMC humanized model
G1. Vehicle, i.p. QW
G2. Drug A, 1 mpk, i.v., single
G3. Drug A, 2 mpk, i.v., single
G4. Drug B, 3 mpk, i.p. QW
G5. Drug A, 1 mpk + Drug B, 3 mpk
G6. Drug A, 2 mpk + Drug B, 3 mpk
Tumor volume (mm3, Mean±SEM)
2000
1500
1000
500
0
0
7
14
21
Time after dosing (days)
****
****
****
****

Figure 1

Calu-6 PBMC humanized model
G1. Vehicle, i.p QW
G2. Drug A, 1 mpk, i.v., single
G3. Drug A, 2 mpk, i.v., single
G7. Drug C, 20 mpk, i.p. QW
G8. Drug A, 2 mpk + Drug C, 20 mpk
Tumor volume (mm3, Mean±SEM)
2000
1500
1000
500
0
0
7
14
21
Time after dosing (days)
****
****
****

Figure 2

SJSA-1 model
Tumor volume (mm$^3$)
Mean ± SEM
3000
2000
1000
0
0 3 6 9 12 15 18 21 24
Time after dosing (days)
Vehicle
Drug A
Drug B
Drug A + Drug B

**Figure 3**

SJSA-1 model
Tumor volume (mm$^3$)
Mean ± SEM
3000
2000
1000
0
0 3 6 9 12 15 18 21 24
Time after dosing (days)
Vehicle
Drug A
Drug C
Drug A + Drug C

**Figure 4**

SJSA-1 model
Change in body weight (%)
Mean ± SEM
20
15
10
5
0
-5
-10
-15
0
3
6
9
12
15
18
21
24
Time after dosing (days)
Vehicle
Drug A
Drug B
Drug C
Drug A + Drug B
Drug A + Drug C

**Figure 5**

**INTERNATIONAL SEARCH REPORT**

International application No.
**PCT/CN2024/125546**

**A. CLASSIFICATION OF SUBJECT MATTER**

A61K39/395(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: A61K, A61P

Documentation searched other than minimum documentation to the extent that such documents are included in the fields searched

Electronic data base consulted during the international search (name of data base and, where practicable, search terms used)

CNTXT, ENTXTC, CNABS, VEN, CNKI, ENTXT, PUBMED, ISI WEB OF SCIENCE, NCBI_GenBank, STN, 中国专利生物序列检索系统, China Patent Biological Sequence Search System: 抗体偶联药物, 抗体药物偶联物, 阿得贝利, 艾瑞卡, 肺癌, 骨肉瘤, 卡瑞利珠, 抗体, 偶联, 依喜替康, 翰森, 豪森, 恒邦, 孙丹妮, 周远锋, 徐敏, 刘军豪, 邱鹏超, ADC, Adebrelimab, B7H3, camrelizumab, exatecan, hanson, Osteosarcoma, Lung Cancer, DX-8951, DX8951, PD-L1, antibody drug conjugate, FADC-2, B7-H3, PD-1, DANNI SUN, YUANFENG ZHOU, SEQ ID NOs: 1-10

**C. DOCUMENTS CONSIDERED TO BE RELEVANT**

| Category* | Citation of document, with indication, where appropriate, of the relevant passages | Relevant to claim No. |
|---|---|---|
| Y | WO 2020063673 A1 (JIANGSU HENGRUI MEDICINE CO., LTD. et al.) 02 April 2020 (2020-04-02)<br>claims 1, 19-20 and 24-28, and description, page 44, paragraphs 3-4 and 6-8, page 46, embodiment 11, and page 54, paragraphs 1-3, and table 9 | 1-27 |
| Y | KR 20210063070 A (LEGOCHEM BIOSCIENCES INC. et al.) 01 June 2021 (2021-06-01)<br>description, paragraphs 510 and 519-520, and embodiments 7-8 | 1-27 |
| Y | WO 2021190586 A1 (JIANGSU HENGRUI MEDICINE CO., LTD. et al.) 30 September 2021 (2021-09-30)<br>page 15, lines 6-14, page 28, lines 10-29, page 48, embodiment 3, and pages 54-57, test examples 2-4 | 1-27 |
| Y | CN 110049779 A (DAIICHI SANKYO COMPANY, LIMITED) 23 July 2019 (2019-07-23)<br>claims 1-92 | 1-27 |

☑ Further documents are listed in the continuation of Box C. ☑ See patent family annex.

* Special categories of cited documents:

"A" document defining the general state of the art which is not considered to be of particular relevance

"D" document cited by the applicant in the international application

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **20 January 2025** | **26 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/125546**

**C. DOCUMENTS CONSIDERED TO BE RELEVANT**

| Category* | Citation of document, with indication, where appropriate, of the relevant passages | Relevant to claim No. |
| --- | --- | --- |
| A | WO 2019024911 A1 (JIANGSU HENGRUI MEDICINE CO., LTD. et al.) 07 February 2019 (2019-02-07)<br>claims 1-22 | 1-27 |
| A | LI, H.Y. et al. "Bispecific Antibody Targeting Both B7-H3 and PD-L1 Exhibits Superior Antitumor Activities"<br>*Acta Pharmacologica Sinica,* Vol. vol. 44, 16 June 2023 (2023-06-16),<br>abstract | 1-27 |

Form PCT/ISA/210 (second sheet) (July 2022)

**INTERNATIONAL SEARCH REPORT**

International application No.
**PCT/CN2024/125546**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☐ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☑ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/125546**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **23-27**
because they relate to subject matter not required to be searched by this Authority, namely:

Claims 23-27 relate to a method for treating cancer, which falls within methods for treatment of diseases, and falls within the cases set out in PCT Rule 39.1(iv) for which no search is required by the International Searching Authority. A search is conducted on the basis that the subject matter thereof is amended to be the subject matter of a corresponding pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/125546**

| Patent document cited in search report | Publication date (day/month/year) | Patent family member(s) | Publication date (day/month/year) |
|---|---|---|---|
| WO 2020063673 A1 | 02 April 2020 | MX 2021003446 A | 15 June 2021 |
| | | JP 2022512568 A | 07 February 2022 |
| | | JP 7408646 B2 | 05 January 2024 |
| | | EP 3854816 A1 | 28 July 2021 |
| | | EP 3854816 A4 | 07 September 2022 |
| | | ZA 202102696 B | 25 October 2023 |
| | | TW 202028242 A | 01 August 2020 |
| | | TWI 846736 B | 01 July 2024 |
| | | CA 3114474 A1 | 02 April 2020 |
| | | AU 2019351427 A1 | 15 April 2021 |
| | | US 2021347894 A1 | 11 November 2021 |
| | | BR 112021004829 A2 | 08 June 2021 |
| | | KR 20210068457 A | 09 June 2021 |
| KR 20210063070 A | 01 June 2021 | None | |
| WO 2021190586 A1 | 30 September 2021 | TW 202144015 A | 01 December 2021 |
| CN 110049779 A | 23 July 2019 | BR 112019011794 A2 | 29 October 2019 |
| | | US 2022143009 A1 | 12 May 2022 |
| | | JP 2022191375 A | 27 December 2022 |
| | | AU 2017377233 A1 | 13 June 2019 |
| | | AU 2017377233 B2 | 19 December 2024 |
| | | JPWO 2018110515 A1 | 24 October 2019 |
| | | WO 2018110515 A1 | 21 June 2018 |
| | | SG 10201912575 RA | 27 February 2020 |
| | | EP 3552626 A1 | 16 October 2019 |
| | | EP 3552626 A4 | 10 June 2020 |
| | | KR 20190095280 A | 14 August 2019 |
| | | TW 201828993 A | 16 August 2018 |
| | | US 2019314362 A1 | 17 October 2019 |
| | | US 11273155 B2 | 15 March 2022 |
| | | JP 2024156920 A | 06 November 2024 |
| | | CA 3046293 A1 | 21 June 2018 |
| WO 2019024911 A1 | 07 February 2019 | TW 201909926 A | 16 March 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014179664 A
- WO 2018085468 A
- WO 2018129559 A
- WO 2020063673 A

### Non-patent literature cited in the description

- **CHOTHIA et al.** *Nature*, 1989, vol. 342, 877-883
- **AL-LAZIKANI et al.** Standard conformations for the canonical structures of immunoglobulins. *Journal of Molecular Biology*, 1997, vol. 273, 927-948
- **KABAT et al.** Sequences of Proteins of Immunological Interest. U.S. Department of Health and Human Services, 1987
- **WARD et al.** *Nature*, 1989, vol. 341, 544-546